Europäisches Patentamt

(19) European Patent Office

Office européen des brevets

(11) EP 0 601 924 B1

(12) **FASCICULE DE BREVET EUROPEEN**

(45) Date de publication et mention
de la délivrance du brevet:
**22.04.1998 Bulletin 1998/17**

(21) Numéro de dépôt: **93402948.9**

(22) Date de dépôt: **07.12.1993**

(51) Int. Cl.6: **C01B 39/02**, B01J 29/70,
C07C 5/22, C07C 4/06,
C10G 50/02, B01J 20/18

(54) **Procédé de synthèse d'un aluminosilicate cristallin enrichi en silice, ayant la structure de la mazzite, l'aluminosilicate obtenu et son utilisation comme catalyseur de transformation des hydrocarbures**

Verfahren zur Synthese eines an Kieselsäure angereicherten, kristallinen Alumosilikates mit Mazzit-Struktur, das erhaltene Alumosilikat sowie seine Verwendung als Katalysator für die Umwandlung von Kohlenwasserstoffen

Process for the synthesis of a crystalline aluminosilicate enriched in silica having the mazzite structure, the aluminosilicate thus obtained and its use as catalyst in the conversion of hydrocarbons

(84) Etats contractants désignés:
**BE DE FR GB IT NL**

(30) Priorité: **08.12.1992 FR 9214774**

(43) Date de publication de la demande:
**15.06.1994 Bulletin 1994/24**

(73) Titulaire: **ELF AQUITAINE**
**92400 Courbevoie (FR)**

(72) Inventeurs:
• **Di Renzo, Francesco**
**F-34080 Montpellier (FR)**
• **Fajula, François**
**F-34100 Montpellier (FR)**
• **Barbouth, Nisso**
**F-92300 Chatillon (FR)**
• **Fitoussi, Fredj**
**F-69007 Lyon (FR)**
• **Schulz, Philippe**
**F-69110 Ste Foix les Lyon (FR)**
• **des Courrieres, Thierry**
**F-69007 Lyon (FR)**

(74) Mandataire: **Boillot, Marc**
**Elf Exploration Production**
**Département Propriété Industrielle**
**Tour Elf**
**EP/T/RD/DPI - Bureau 34 G 47**
**92078 Paris La Défense Cedex (FR)**

(56) Documents cités:
EP-A- 0 419 307          DE-A- 1 965 437
FR-A- 2 584 385          GB-A- 2 160 517
US-A- 3 642 434

## Description

La présente invention concerne la synthèse d'un aluminosilicate cristallin enrichi en silice, ayant la structure cristalline de la mazzite, l'aluminosilicate ainsi obtenu et son utilisation comme catalyseur de transformation des hydrocarbures.

Obtenue par voie de synthèse hydrothermale pour la première fois en 1966 sous le nom de zéolithe oméga par Flanigen et Kellberg (U.S.4241036), la mazzite est un aluminosilicate naturel identifié en 1972 dans les roches basaltiques du Mont Semiol, près de Montbrison, Loire, France.

La structure de la mazzite a été résolue par Galli (Cryst. Structure Comm. 3, 339, 1974) et Rinaldi et coll. (Acta Cryst. B31, 1603, 1975). La structure, de symétrie hexagonale, résulte de l'empilement de cages gmélinites (polyèdres à 14 faces) qui mettent en commun leurs faces constituées d'anneaux à 6 tétraèdres selon l'axe cristallographique c. Les colonnes de cages gmélinites sont décalées de c/2. Le réseau tridimensionnel qui résulte de cet assemblage crée trois systèmes de canaux, tous parallèles à l'axe c:

i) Des canaux quasi cylindriques, délimités par des pores de 12 tétraèdres dont le diamètre libre est de 0,74 nm (1 nm = $10^{-9}$ m). Les parois de ces canaux sont constituées de chaînes infinies de cycles à quatre et cinq tétraèdres et sont donc impénétrables par la plupart des molécules usuelles.
ii) Les colonnes de cages gmélinites, dont l'accès est très limité, au travers des cycles à 6 tétraèdres de diamètre libre de 0,28 nm.
iii) Des canaux constitués par les intervalles entre les colonnes de cages gmélinites, délimités par des cycles à 8 tétraèdres déformés d'un diamètre libre de 0,34 nm.

Du fait de sa structure, la mazzite appartient donc à la catégorie des zéolithes à grands pores à porosité unidirectionnelle. Ses propriétés de tamis moléculaire sont décrites dans plusieurs publications et brevets (Flanigen, B.F. 1 548 382, Breck et coll. Adv. Chem. Ser. 121, 319, 1973, Ciric, B.F. 1 502 289, Perrotta et coll., J. Catal. 55, 249, 1978, Chauvin et coll., J. Catal. 11, 94, 1988).

Du fait de leur grand diamètre de pores et de leur forte acidité, les zéolithes de la famille de la mazzite ont suscité un vif intérêt pour des applications en catalyse comme le craquage (Perrotta et coll., J. Catal 55, 249, 1978) et l'hydrocraquage (Cole et coll. Adv. Chem. Ser. 121, 583, 1973) des gazoles, l'alkylation (Bowes et coll. U.S. 3 578 728, Flanigen et coll. U.S. 4241036) et l'isomérisation (Solinas et coll. Appl. Catal 5, 171, 1983) des aromatiques, l'hydratation des oléfines (Fajula et coll. J. Catal. 89, 64, 1984), l'isomérisation des parafines légères (Raatz et coll., B.F. 2657869) et l'hydroconversion des alcanes (Chauvin et coll. Stud. Surf. Sci. Catal. 49B, 1397, 1989).

De nombreux protocoles expérimentaux sont connus à ce jour pour la synthèse des zéolithes de type mazzite. Les zéolithes de cette famille (oméga, ZSM-4, LZ 202, MZ-34) sont préparées par réaction hydrothermale entre 90 et 150°C de gels aluminosilicatés alcalins. La cristallisation est généralement effectuée en présence de cations sodium et d'un agent structurant organique comme le tétraméthylammonium ou la choline par exemple. Un brevet (Cannan U.S. 4840779) décrit une synthèse de mazzite en absence d'agent organique où la cristallisation est initiée par l'addition de germes de zéolithe dans le milieu de synthèse. Dans un autre exemple (Di Renzo et coll. B.F. 2651221) la synthèse est effectuée en présence d'ions sodium, potassium et tétraméthylammonium. Les sources de silicium sont celles usuellement employées par l'homme du métier, à savoir les gels de silice, les silices colloïdales, les silices précipitées, les silicates, les esters siliciques hydrolysables. De même, les sources d'aluminum sont celles connues dans l'art comme les aluminates, les hydroxydes, les alumines ou les aluminosilicates amorphes et cristallisés. L'utilisation d'aluminosilicates cristallisés, naturels ou synthétiques, dans la synthèse des zéolithes est notamment utilisée pour réguler la croissance cristalline au travers d'une dissolution lente et régulière de la source d'aluminium. En règle générale, on utilise, comme source d'aluminium cristalline, un matériau dont la stabilité (solubilité), dans le milieu de synthèse, est inférieure à celle de la zéolithe que l'on veut obtenir. La croissance de la zéolithe désirée entraîne la dissolution de la source d'aluminium. Parmi les exemples de cristallisation de zéolithes de type mazzite à partir d'aluminosilicates, on peut citer les synthèses à partir d'argiles naturelles (Dwyer et coll. U.S. 4.091.007, Fajula et coll. U.S. 4891200) ou de zéolithes naturelles et synthétiques de type A, X, Y, ERI, MOR (Dwyer U.S. 3.642.434, Plank, B.F. 2027390).

La transformation d'une phase aluminosilicate cristalline en une autre dans des conditions hydrothermiques est un phénomène bien connu par l'homme de l'art. Cette transformation peut éventuellement intervenir sans que l'on introduise délibérément une source de réactif cristalline dans le milieu. Il n'est pas rare en effet que l'apparition de la phase zéolithique souhaitée soit précédée de la formation transitoire d'une autre phase, moins stable dans le milieu (voir par exemple R.M. Barrer, dans "Hydrothermal Chemistry of zeolites", Academic Press London, pp 174-176, 1982). Ces transformations sont conformes à la règle d'Ostwald qui stipule que, dans un système où plusieurs polymorphes peuvent se former, le système évolue vers l'état le plus stable thermodynamiquement en passant par l'intermédiaire d'états métastables. L'évolution de la silice amorphe en cristobalite puis en keatite et enfin en quartz obéit à cette règle (Car et coll. Amer. Mineral. 43, 908, 1958). Dans le cas des zéolithes les transformations amorphe > faujasite (Y) > gismondine (P), en présence d'ions sodium, et amorphe > faujasite (Y) > mazzite, en présence d'ions tétraméthylammonium, sont des exemples classiques (Dwyer et coll. J. Catal. 50, 263, 1979, Fajula et coll. Zeolites, 7, 203, 1987).

Quelque soit le mode de synthèse employé, les précurseurs de zéolithe mazzite, c'est-à-dire les produits bruts de synthèse, sont obtenus avec des rapports Si/Al compris entre 2,5 et 5 (soit des fractions molaires d'aluminium $m = Al/(Al + Si)$ de 0,285 à 0,166). Cette haute teneur en aluminium, couplée à une porosité unidirectionnelle a des conséquences néfastes pour les applications en catalyse. D'une part, lors de l'activation thermique, destinée à décomposer l'agent organique piégé dans la structure, il se produit une dégradation partielle de la structure qui entraîne la formation de résidus amorphes restant piégés dans les pores. Ces résidus obstruent les canaux, ce qui conduit à un blocage sévère de la porosité. D'autre part, la forte densité de centres polaires, associés aux atomes d'aluminium, favorise les réactions de cokage lors des réactions catalytiques provoquant une désactivation rapide des catalyseurs.

Afin de pallier ces inconvénients, on effectue généralement une désalumination contrôlée de la zéolithe avant son emploi en catalyse. Cette désalumination a pour objet d'adapter l'acidité de la zéolithe aux exigences de la réaction. Il est connu que pour chaque zéolithe et pour chaque réaction catalysée par elle, la fraction molaire aluminique m joue un rôle fondamental. Elle détermine directement la densité et la force des centres acides. Parmi les méthodes classiques de désalumination, celles s'effectuant par substitution isomorphe d'atomes d'aluminium par des atomes de silicium, par exemple les traitements à l'hexafluorosilicate d'ammonium, (Breck U.S. 4503023) ou au tétrachlorure de silicium. (Beyer et coll. Stud. Surf. Sci. Catal. 24, 263, 1985) ne conduisent pas à des résultats satisfaisants dans le cas de structures à porosité unidirectionnelle car elles produisent une désalumination limitée et irreproductible du fait des contraintes diffusionnelles imposées par la structure. De plus, même dans les cas où la désalumination par cette voie est effectuée avec succès, la porosité de la zéolithe n'est pas modifiée par le traitement et on n'observe pas de gain de stabilité de l'activité catalytique après désalumination (Chauvin et coll. J. Catal. 126, 532, 1990).

Une méthode de désalumination permettant à la fois un ajustement de la teneur en aluminium du réseau et la création d'un système de pores secondaires, bénéfique à la diffusion des réactifs et produits, tout en préservant la cristallinité, consiste à effectuer un traitement hydrothermique de la zéolithe suivi d'une attaque acide, cette seconde étape étant destinée à dissoudre et éliminer les résidus extraits de la charpente zéolithique. La littérature fournit plusieurs exemples de désalumination de zéolithes de type mazzite par cette voie (Raatz B.F. 2584 385, Chauvin et coll. Zeolites, 10, 174, 1990, Massiani et coll. Appl. Catal. 42, 105, 1988). Le traitement hydrothermique est effectué à une température comprise entre 400 et 800°C, soit en balayant le solide avec un gaz humide soit en immergeant la zéolithe hydratée dans un four préchauffé (steaming et self steaming respectivement, en employant la terminologie anglosaxonne). L'attaque acide est réalisée en présence d'une solution aqueuse d'acide minéral, avec une concentration comprise entre 0,01 et 10 N à une température comprise entre 20 et 150°C.

Si le taux d'extraction des atomes d'aluminium du réseau de la zéolithe par traitement hydrothermique peut être assez bien contrôlé par le biais des conditions opératoires (pression d'eau, température, temps), il n'en est pas de même pour ce qui est de l'attaque acide. Cette difficulté est probablement liée au fait que les réactions de polymérisation ou condensation des espèces aluminiques extraites du réseau et qui interviennent à l'intérieur des pores de la zéolithe ne sont pas bien maîtrisées. Le traitement hydrothermique conduit ainsi à la formation d'espèces de nature chimique et de taille, donc de réactivité (solubilité) vis-à-vis de l'acide, très différentes.

Une attaque acide trop sévère s'accompagne d'une désalumination supplémentaire du réseau, voire d'une dissolution partielle de celui-ci, et d'une perte de critallinité pouvant aller jusqu'à l'amorphisation complète de la structure. A l'inverse, si la quantité d'acide, le temps ou la température de réaction ne sont pas suffisants, le solide final contient des résidus occlus dans les pores qui limitent l'accès des réactifs et contribuent à la formation de coke lors des réactions catalytiques. Les conditions de l'attaque acide sont donc difficiles à établir à priori car elles dépendent elles-mêmes de la taille des cristaux, du taux de désalumination, de la nature des espèces hors réseau, de la teneur en cations résiduels et, probablement, d'autres facteurs non encore appréhendés en l'état actuel des connaissances. De ce fait, la désalumination par voie hydrothermique et attaque acide demeure encore empirique. La préparation de solides désaluminés cristallins, avec une porosité accessible et libres de résidus aluminosilicatés hors réseau nécessite plusieurs étapes successives d'échange cationique, de traitement à la vapeur d'eau et d'attaque acide. La multiplication de ces étapes élémentaires rend aléatoire et en tout état de cause extrèmement coûteuse une industrialisation de ce procédé pour la désalumination de zéolithes de type mazzite.

Nous avons trouvé maintenant un procédé simplifié qui permet d'obtenir en peu d'étapes un aluminosilicate cristallin, de fraction molaire en aluminium faible, ayant la structure de la mazzite.

Cet aluminosilicate présente une bonne activité et sélectivité dans les réactions de catalyse acide et se caractérise par une porosité facilement accessible, libre de tout débris aluminosilicaté.

Notre procédé de préparation d'un aluminosilate cristallin enrichi en silice ayant la structure de la mazzite comporte les étapes suivantes :

- préparation d'un gel de cristallisation renfermant au moins une source de silicium tétravalent, d'aluminium trivalent et d'ion alcalin, un structurant organique et de l'eau ;

- chauffage du gel pour obtenir la cristallisation, puis séparation, lavage et séchage des cristaux ;
- traitement thermique sous atmosphère humide ;
- puis attaque par un acide minéral
- et il est caractérisé en ce que la source d'aluminium trivalent est constituée de cristaux de zéolithe Y apparaissant sous forme de sphères ou de botryoïdes.

Les grains de zéolithe Y qui conviennent pour le procédé selon l'invention sont donc délimités uniquement par des surfaces arrondies et non pas par des faces planes.

Il peut être avantageux de soumettre les cristaux séchés, avant le traitement thermique sous atmosphère humide, appelé traitement hydrothermique, à une calcination à une température suffisante pour décomposer le structurant organique. Après cette calcination et avant le traitement hydrothermique, il est possible d'échanger des ions alcalins présents dans les cristaux par des protons ou des ions ammonium.

La composition molaire globale du gel de cristallisation doit rester de préférence dans les limites :

$SiO_2/Al_2O_3 = 20-200$
$SiO_2$/ion alcalin = 1,2-2
structurant/ion alcalin = 0,01-0,1
$H_2O$/ion alcalin = 15-100

Comme ion alcalin on utilise en général du sodium ou un mélange de sodium et de potassium.

Parmi les structurants organiques les tétraalkylammoniums sont plus couramment utilisés et de préférence le tétraméthylammonium.

La source de silicium tétravalent peut être choisie parmi les silicates, les silices solides, les silices colloïdales, les gels et xerogels, les esters siliciques hydrolysables ou les diatomites.

La zéolithe de type Y, de morphologie appropriée, est avantageusement préparée par cristallisation à 50°C pendant dix jours sous agitation d'un mélange alcalin d'aluminosilicate de composition molaire globale :

a $Na_2O$, $Al_2O_3$ ; b $SiO_2$ , c $H_2O$ où a est compris entre 2 et 6, b entre 5 et 15 et c entre 100 et 200.

L'alcalinité et la source de sodium sont généralement fournies par la soude. La source de silicium peut être de même origine que pour la synthèse du précurseur de mazzite. La source d'aluminium est en général un sel d'aluminium comme l'aluminate de sodium ou un hydroxyde d'aluminium réactif. Les cristaux de zéolithe Y sont récupérés par filtration ou centrifugation, lavés à l'eau désionisée et éventuellement séchés à l'étuve entre 50 et 100°C.

Le précurseur de mazzite est cristallisé à une température comprise entre 20 et 200°C, de préférence entre 100 et 150°C sous une pression au moins égale à la tension de vapeur du gel, pendant 4 à 96 heures et de préférence de 10 à 50 heures.

Les cristaux de mazzite sont séparés du milieu réactionnel par filtration ou centrifugation, lavés et séchés à l'étuve entre 50 et 100°C.

La calcination destinée à décomposer l'agent organique occlus dans les pores s'effectue sous un flux d'azote ou d'air ou d'un mélange des deux gaz. Elle consiste en un chauffage pendant 5 minutes à 10 heures à une température supérieure à 450°C.

Pour l'échange des alcalins on utilise une solution aqueuse d'un sel d'ammonium ou un acide minéral ou organique à une température comprise entre 20 et 90°C. Le taux pondéral résiduel de sodium après échange est généralement inférieur à 1 %.

Le traitement thermique sous atmosphère humide, appelé traitement hydrothermique, est effectué à une température comprise entre 450 et 850°C sous une atmosphère humide pendant une durée de 1 minute à quelques heures. Les paramètres peuvent être ajustés de manière à obtenir le rapport Si/Al visé dans le réseau tridimensionnel de zéolithe $(Si/Al)_R$.

Pour l'attaque par l'acide minéral on utilise en général un acide tel que l'acide nitrique, l'acide chlorhydrique ou l'acide sulfurique. Un choix judicieux de la concentration en acide, de la durée et de la température du traitement permettent d'éliminer quantitativement les débris aluminosiliciques sans affecter la cristallinité. En général, la durée de l'attaque acide est comprise entre 10 minutes et 5 heures, la température entre 20 et 100°C et la concentration de l'acide entre 0,01 et 10 N.

L'aluminosilicate obtenu présente un diffractogramme des rayons X caractéristique de la mazzite sans fond continu pouvant traduire la présence de phase amorphe. Son volume poreux, mesuré par adsorption d'azote à 77 K, est compris entre 0,1 et 0,4 ml/g avec de 5 à 50 % de celui-ci contenu dans des mésopores (pores d'un diamètre compris entre 2 et 50 nm). Sa teneur en aluminium global, mesurée par analyse élémentaire, est identique à celle de la charpente zéolithique. La teneur en aluminium dans le réseau est aisément mesurable par l'homme de l'art grâce à l'utilisation de méthodes spectroscopiques telles que la spectroscopie de RMN haute résolution du $^{29}$Si (voir par exemple Engelhardt et coll. "High-Resolution Solid-State NMR of Silicates and Zeolites", John Wiley and Sons, 1987), la spectroscopie infra rouge ou la diffraction des rayons X (thèse B. Chauvin, Montpellier, 1988). La méthode la plus directe cependant pour la mise en évidence de la présence de résidus aluminosiliciques hors réseau est la spectroscopie RMN haute résolution de $^{27}$Al. Il est bien connu en effet que les atomes d'aluminium de la charpente zéolithique ont tous un environnement tétraédrique (Td) régulier alors que les atomes d'aluminium associés à des résidus hors réseau, ou débris silicoaluminés, présentent une coordination 6 (symétrie octaédrique) ou, éventuellement, 5. Chacune de ces coordinations de l'aluminium conduit à des signaux caractéristiques et facilement discernables dans les spectres RMN haute résolution de $^{27}$Al. Ainsi, les atomes d'aluminium du réseau dans une coordination tétraédrique donnent des signaux dans les domaines des déplacements chimiques compris entre 50 et 65 ppm (parties par million par rapport à une référence comme un sel ou hydroxyde d'aluminium en solution aqueuse). La position exacte et le nombre des signaux des atomes tétraédriques dépend du nombre de sites cristallographiques non-équivalents du réseau. Ceux-ci sont au nombre de deux dans la mazzite. Les atomes d'aluminium hexacoordinés (hors réseau) donnent un signal entre 5 et -5 ppm.

Enfin les atomes d'aluminium, dans une coordination 5, donnent un signal entre 20 et 40 ppm.

Les aluminosilicates cristallins enrichis en silice conformes à la présente invention possèdent des rapports Si/Al de réseau $(Si/Al)_R$ et globaux $(Si/Al)_G$ identiques, supérieurs à 10, et se caractérisent par des spectres de RMN haute résolution de $^{27}$Al ne contenant que les signaux dûs aux atomes d'aluminium tétracoordinés du réseau critallin, donc compris entre 50 et 65 ppm.

Sans vouloir être limités par aucune considération théorique, nous pensons que l'élimination totale des débris silicoaluminés hors réseau lors de l'attaque acide est rendue possible par une répartition particulière des atomes d'aluminium dans le précurseur. Au cours du traitement hydrothermique les espèces extraites du réseau demeureraient divisées, donc réactives et facilement solubilisables par l'acide.

La mazzite enrichie en silice obtenue peut être utilisée comme catalyseur, pur, sous forme de poudre ou de grains. Il peut être avantageux d'y adjoindre un liant inerte comme l'alumine, la silice, la silice-alumine ou des argiles, particulièrement pour la préparation d'extrudés ou de billes.

Il est également possible d'utiliser la mazzite enrichie en silice en combinaison avec d'autres catalyseurs ou phases actives. Les autres catalyseurs peuvent être des zéolithes, des argiles, des métaux ou des oxides, en mélange mécanique ou incorporés dans le même liant que la mazzite. Parmi les phases actives que l'on peut ajouter à la mazzite désaluminée on citera les cations de terres rares, les oxides finement divisés ou les métaux nobles. Dans ce cas la phase active additionnelle peut être incorporée par toute méthode connue dans l'art, comme le dépôt en surface, l'imprégnation ou l'échange cationique, effectuée soit sur la poudre de zéolithe pure soit sur la zéolithe mise en forme.

Les catalyseurs obtenus sont utilisables, seuls ou associés à d'autres catalyseurs, pour les réactions de transformation des hydrocarbures comme l'isomérisation des alcanes, des oléfines et des aromatiques, le craquage et l'hydrocraquage des gazoles et des résidus, la polymérisation des oléfines légères pour la synthèse de lubrifiants ou d'autres réactions de transformation des fractions pétrolières.

La mazzite désaluminée est également utile pour la transformation des substrats organiques et comme tamis moléculaire pour les opérations de séchage et de séparation.

Les exemples suivants illustrent l'invention sans toutefois la limiter.

## EXEMPLE 1

a) On prépare une zéolithe de type Y par critallisation, pendant 10 jours à 50°C sous agitation, d'un mélange de composition

4 $Na_2O$ ; $Al_2O3$ ; 9,2 $SiO_2$ ; 155 $H_2O$

obtenu à partir de 28,4 grammes de soude en pastilles, 320,1 g d'eau, 19,4 g d'aluminate de sodium ($NaAlO_2$) et 70,8 g de silice en poudre. Le solide récupéré (50 g) lavé et séché présente un diffractogramme des rayons X caractéristique de la zéolithe Y. Son aspect, examiné par microscopie électronique à balayage, est celui de grains à la surface arrondie de 0,2 à 0,5 $\mu$m de diamètre. Son rapport Si/Al déterminé par analyse élémentaire est de 2,7.

b) Le précurseur de mazzite est obtenu par critallisation d'un mélange

5,2 $Na_2O$ ; 0,36 $TMA_2O$ ; $Al_2O_3$ ; 15 $SiO_2$ ; 393 $H_2O$

préparé comme suit :

On dissout 66,1 g de soude en pastilles et 14,2 g de chlorure de tétraméthylammonium dans 1004,8 g d'eau. Après refroidissement de la solution, on rajoute 149,6 g de silice (gel de silice, CECAGEL de CECA) sous forte agitation. Après 10 minutes, on rajoute une suspension de 87,6 g de zéolithe Y obtenue en a) dans 200 ml d'eau. Le gel est maintenu sous agitation pendant deux heures à température ambiante puis transvasé dans un autoclave en acier de 2 1. L'autoclave est chauffé à 115°C et maintenu à cette température pendant 24 heures avec une vitesse d'agitation du mélange de 150 tours par minute.

Les cristaux, récupérés par filtration, lavés et séchés à l'étuve à 110°C conduisent à un difractogramme des rayons X caractéristique de la mazzite pure. Leur taille est de 1 à 1,5 $\mu$m de long pour 0,5 à 0,7 $\mu$m de large (longueur sur diamètre égal à 2) avec un faciès de prisme hexagonal régulier. L'analyse élémentaire conduit à un rapport Si/Al de 4

$$\text{(fraction aluminique } m = \frac{Al}{Al + Si} = 0,20)$$

Le précurseur de mazzite a été activé par :

- calcination sous flux d'air à 550°C pendant 4 heures pour décomposer l'agent organique,
- échange des ions sodium résiduels par une solution de nitrate d'ammonium 0,5 N à 80°C,
- traitement sous une atmosphère de vapeur d'eau à 620°C pendant deux heures,
- attaque acide pendant 4 heures à la température de reflux avec une solution 1,5 N d'acide nitrique et un rapport volume de solution (ml) /poids de zéolithe (g) de 10.

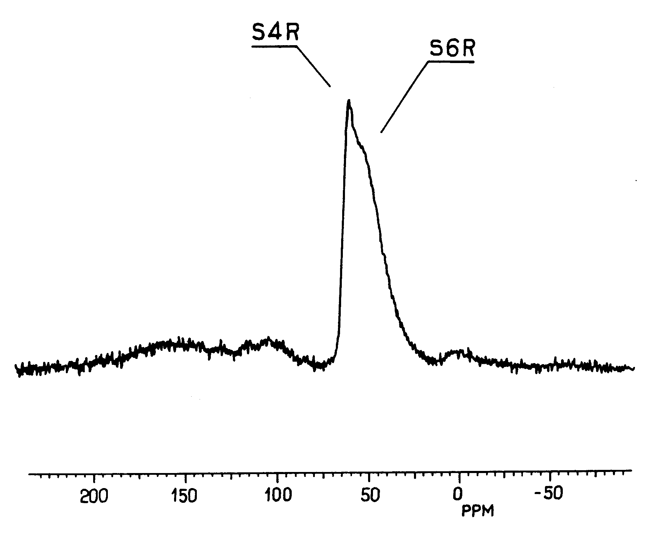

A l'issue du traitement de désalumination la zéolithe présente une cristallinité supérieure à 90 % (évaluée par diffraction des rayons X). L'analyse élémentaire conduit à un rapport Si/Al$_G$ de 22 (m = 0,043) et une teneur en sodium résiduel inférieure à 0,05 % poids. Les analyses par RMN du $^{29}$Si et par spectroscopie infrarouge révèlent un rapport Si/Al$_R$ de 22 c'est-à-dire identique à celui trouvé par analyse élémentaire. Ce résultat qui traduit l'absence d'espèces aluminosilicates occluses dans la porosité a été confirmé par l'analyse RMN de l'aluminium 27. Le spectre obtenu, rapporté dans la figure 1, montre de manière univoque que les seuls atomes d'aluminium contenus dans le solide correspondent aux atomes du réseau dans les deux sites cristallographiques (cycles à 4, signal à 61 ppm (S4R), et cycles à 6, signal à 54 ppm (S6R)).

## EXEMPLE 2

Une zéolithe de type Y est préparée par cristallisation à 50°C pendant 12 jours sans agitation d'un mélange :
4 Na$_2$O ; Al$_2$O$_3$ ; 9,9 SiO$_2$ ; 153 H$_2$O
préparé à partir de 160 g d'eau, 14,2 g de soude, 7 g d'aluminate de sodium et 35,5 g de silice solide.

Cette zéolithe Y possède un rapport Si/Al de 2,34 et Na/Al de 1. Ses particules consistent en des agregats de cristaux arrondis de 0,25 $\mu$m de diamètre moyen.

Le précurseur de mazzite est synthétisé dans un autoclave en acier de 900 ml à partir d'un mélange
20,6 Na$_2$O ; 1,2 TMA$_2$O ; Al$_2$O$_3$ ; 60 SiO$_2$ ; 1455 H$_2$O
préparé à partir de 500 g d'eau, 31,45 g de soude, 81 g de gel de silice (CECAGEL) 18,68 g de zéolithe Y et 9,05 g d'hydroxyde de tétraméthyammonium pentahydrate. Ce mélange est cristallisé à 115°C pendant 24 heures sous agitation (300 tours/mn). Le solide récupéré correspond à de la mazzite pure apparaissant sous forme de cristaux hexagonaux d'une taille moyenne 1,5 x 0,7 $\mu$m et de rapport Si/Al de 5.

Un gramme de ce lot de mazzite a été activé comme indiqué dans l'exemple 1 excepté que la température du traitement hydrothermique a été de 600°C et la concentration en acide de 1 N. Les résultats des caractéristiques de ce solide après désalumination conduisent à une cristallinité de 95 %, un rapport Si/Al global et de réseau identique et égal à 15 et un spectre de RMN de $^{27}$Al ne comportant que les signaux à 60 ± 2 et 54 ± 2 ppm des atomes de la charpente zéolithique.

## EXEMPLE 3 (Comparatif)

Le protocole opératoire de l'exemple 1 ci-dessus a été utilisé pour préparer un précurseur de mazzite en utilisant une zéolithe Y commerciale (NaY de ZEOCAT) de rapport Si/Al égal à 2,7 et dont les cristaux sont constitués de particules polygonisés (cristaux mâclés exposant des faces à bas indice de Miller) de 0,4 à 1 $\mu$m en taille.

Après cristallisation pendant 24 heures à 115°C sous agitation le solide obtenu correspond à un mélange de 43 % de sodalite, 52 % d'analcime et 5 % seulement de mazzite.

## EXEMPLE 4 (comparatif)

Une mazzite désaluminée a été préparée à partir d'un aluminosilicate à stabilité thermique élevée, obtenue selon l'exemplaire 5 du brevet français 2582234 (brevet américain 4891200). Les cristaux obtenus ont un faciès hexagonal de 3 à 4 $\mu$m de long pour 0,3 à 0,7 $\mu$m de diamètre et un rapport Si/Al de 3,2.

Ce précurseur a été soumis au même traitement désaluminant que celui décrit dans l'exemple 1 ci-dessus. L'analyse élémentaire du solide conduit à un rapport Si/Al global de 17 alors que les mesures par spectroscopie RMN $^{29}$Si

EP 0 601 924 B1

et infrarouge conduisent à un rapport de réseau égal à 22. Dans le spectre de RMN $^{27}$Al la présence d'aluminium hors réseau avec une coordination 6 (signal à 0 ppm) est clairement observable. Cet exemple montre que, bien que le traitement hydrothermique ait conduit à un même taux d'extraction des atomes d'aluminium de la charpente zéolithique, l'attaque acide ultérieure n'a pas permis l'élimination totale des espèces occluses. Une partie de cet échantillon a été utilisée pour préparer les catalyseurs B1 et B2 dont le mode de préparation et les performances sont décrits dans les exemples 5 et 6 ci-après. Une autre portion a été soumise à une nouvelle attaque acide en utilisant une concentration en acide de 2 N. Cette seconde attaque acide a entraîné une importante dégradation de la structure car la fraction recueillie ne présentait qu'une cristallinité de 20 à 30 % par rapport au solide initial.

**EXEMPLE 5**

Préparation des catalyseurs A1 (zéolithe désaluminée selon la procédure décrite dans l'exemple 1, catalyseur conforme à l'invention) et B1 (zéolithe désaluminée selon la procédure décrite dans l'exemple 4, comparatif) et évaluation de leurs performances dans le craquage du n-hexane.

Les catalyseurs A1 et B1 sont préparés par activation thermique à 550°C sous flux d'air de la zéolithe désaluminée pure, sous forme de poudre. La réaction de craquage du n-hexane est effectuée à 350°C, sous pression atmosphérique avec un débit de n-hexane de 1,5 ml/h et un rapport de pressions p($N_2$)/P(n-$C_6$) de 6,2. Le flux sortant du réacteur est régulièrement analysé afin d'évaluer l'activité du catalyseur et la stabilité de cette activité en fonction du temps de travail. Les résultats portés dans le tableau 1 ci-dessous montrent que les catalyseurs conformes à l'invention (catalyseur A1, mazzite désaluminée ne contenant pas de débris silicoaluminés) sont plus actifs et stables que les catalyseurs obtenus par désalumination de mazzites obtenues selon les méthodes de l'art antérieur.

Tableau 1

| Conversion (exprimée en pourcentage de moles d'hydrocarbure craquées) du n-hexane à 350°C. | | |
| --- | --- | --- |
| Temps sous flux (mn) | Conversions (%) | |
| | Catalyseur A1 | Catalyseur B1 (comparatif) |
| 8 | 61,9 | 41,6 |
| 18 | 35,1 | 24,4 |
| 28 | 26,8 | 19,3 |
| 38 | 22,8 | 16,0 |
| 48 | 18,8 | 13,8 |
| 58 | 16,7 | 11,9 |
| 68 | 14,9 | 10,6 |

**EXEMPLE 6**

Préparation d'un catalyseur d'hydroconversion des paraffines légères conforme à l'invention (catalyseur A2 préparé à partir de la zéolithe de l'exemple 1) et exemple comparatif (catalyseur B2 préparé à partir de la zéolithe de l'exemple 4).

La zéolithe désaluminée est malaxée avec l'alumine et de l'eau pour former un mélange pâteux contenant 20 % en poids d'alumine. Ce mélange est extrudé au travers d'une filière, séché et calciné. Les extrudés obtenus ont un diamètre de 1,6mm et une longeur comprise entre 3 et 5 mm.

Le dépôt de platine sur le catalyseur est effectué par échange cationique avec le sel Pt($NH_3$)$_4$Cl$_2$,$H_2O$, en présence d'un ion compétiteur (nitrate d'ammonium). La teneur pondérale en platine des catalyseurs A2 et B2 est de 0,3 %.

Le catalyseur est ensuite activé en effectuant tout d'abord une calcination sous air à 520°C puis une réduction du métal sous flux d'hydrogène en augmentant progressivement la température de 150 à 450°C.

Cette procédure conduit à des phases métalliques parfaitement divisées et réparties à l'intérieur du solide.

L'activité des catalyseurs est évaluée dans une unité catalytique en lit fixe, entre 150 et 300°C, sous 1 atmosphère de pression totale, un rapport molaire $H_2$/n-$C_6$ de 70 et une vitesse spatiale, exprimée par le rapport des masses de charge et de catalyseur, de 0,2 h$^{-1}$.

EP 0 601 924 B1

Les performances des catalyseurs sont définies à partir des critères suivants :

**i)** La température ($T_{50\%}$ en °C) nécessaire pour obtenir une conversion du n-hexane de 50 %. La conversion est définie par : (masse de n-hexane dans la charge-masse de n-hexane dans la recette/masse de n-hexane dans la charge) x 100.
**ii)** Les rendements en isomères dibranchés (en %) à 50% de conversion : Diméthyl 2,3 butane (23DMB) et dimethyl 2,2 butane (22 DMB). Le rendement est calculé par : (masse de l'hydrocarbure considéré/masse totale des hydrocarbures de la recette) x 100.
**iii)** La température d'apparition des produits de craquage ($^{T}$craq. en °C).
**iiii)** La conversion optimum et la température à laquelle cette conversion est atteinte. ($^{\%}$max à T°C).

Les résultats de l'évaluation des deux catalyseurs sont donnés dans le tableau 2 ci-dessous.

Tableau 2

| Evaluation des performances des catalyseurs A2 et B2 pour l'hydroisomérisation d'une charge de n-hexane : | | | | | | |
|---|---|---|---|---|---|---|
| Catalyseur | $^{T}$50% (°C) | Rendement (%) | | $^{T}$craq (°C) | $^{\%}$max à T°C | |
| | | 22DMB | 23DMB | | | |
| A2 | 190 | 5,2 | 8,02 | 232 | 85 | 245 |
| B2 | 200 | 4,9 | 7,32 | 231 | 86 | 255 |

Les données du tableau 2 montrent que le catalyseur A2, conforme à l'invention, est plus actif (températures de travail, $T_{50\%}$ et $\%_{max}$ à T°C, inférieures pour obtenir le même taux de conversion du n-hexane, (points i et iii) mais surtout beaucoup plus sélectif en isomères dibranchés (point ii) que le catalyseur de référence B2 préparé selon les méthodes antérieures de l'art.

**Revendications**

1. Procédé de préparation d'un aluminosilicate cristallin enrichi en silice ayant la structure de la mazzite consistant à

   - préparer un gel de cristallisation renfermant une source de silicium tétravalent, d'aluminium trivalent et d'ion alcalin, un structurant organique et de l'eau;
   - chauffer le gel pour obtenir la cristallisation, puis séparer, laver et sécher les cristaux ;
   - soumettre les cristaux à un traitement thermique sous atmosphère humide ;
   - puis à une attaque par un acide minéral ;
   caractérisé en ce que la source d'aluminium trivalent est constituée de cristaux de zéolithe Y apparaissant sous forme de sphères ou de botryoïdes.

2. Procédé selon la revendication 1 caractérisé en ce que la composition globale du gel de cristallisation correspond à :

   $SiO_2/Al_2O_3$ = 20 - 200
   $SiO_2$/ion alcalin = 1,2 - 2
   structurant/ion alcalin = 0,01 - 0,1
   $H_2O$/ion alcalin = 15 - 100

3. Procédé selon la revendication 1 ou 2 caractérisé en ce que avant le traitement thermique sous atmosphère humide les cristaux sont soumis à une calcination à une température suffisante pour décomposer le structurant organique.

4. Procédé selon l'une des revendications 1 à 3 caractérisé en ce que avant le traitement thermique sous atmosphère humide les cations alcalins présents dans les cristaux sont échangés par des protons ou des ions ammonium.

5. Procédé selon l'une des revendications 1 à 4 caractérisé en ce que la source de silicium tétravalent est choisie parmi les silicates, les silices solides, les silices colloïdales, les gels et xérogels, les esters siliciques hydrolysables ou les diatomites.

6. Procédé selon l'une des revendications 1 à 5 caractérisé en ce que l'ion alcalin est le sodium ou un mélange de sodium et potassium.

7. Procédé selon l'une des revendications 1 à 6 caractérisé en ce que le structurant organique est un tétralkylammonium et de préférence le tétraméthylammonium.

8. Procédé selon l'une des revendications 1 à 7 caractérisé en ce que le gel de cristallisation est chauffé à une température comprise entre 20 et 200°C, de préférence entre 100 et 150°C sous une pression au moins égale à la tension de vapeur du gel pendant 4 à 96 heures et de préférence de 10 à 50 heures.

9. Procédé selon la revendication 3 caractérisé en ce que la calcination s'effectue pendant 5 minutes à 10 heures à une température supérieure à 450°C sous un flux d'azote ou d'air ou leur mélange.

10. Procédé selon la revendication 4 caractérisé en ce que les cations alcalins sont échangés par une solution aqueuse d'un sel d'ammonium ou un acide minéral ou organique.

11. Procédé selon l'une des revendications 1 à 10 caractérisé en ce que le traitement thermique sous atmosphère humide est effectué à une température comprise entre 450 et 850°C pendant une durée de 1 minute à quelques heures.

12. Procédé selon l'une des révendications 7 à 11 caractérisé en ce que pour l'attaque par l'acide minéral on utilise l'acide nitrique, l'acide chlorhydrique ou l'acide sulfurique

13. Procédé selon la revendication 12 caractérisé en ce que la durée de l'attaque acide est comprise entre 10 minutes et 5 heures, la température entre 20 et 100°C et la concentration de l'acide entre 0,01 et 10 N.

14. Procédé selon l'une des revendications 1 à 13 caractérisé en ce que la zéolithe Y sous forme de sphères est préparée par cristallisation hydrothermale d'un mélange renfermant une source de silicium tétravalent, une source d'aluminium trivalent, de la
soude et de l'eau selon les proportions : a $Na_2O$ ; $Al_2O_3$ ; b $SiO_2$ ; C $H_2O$ où a est compris entre 2 et 6, b est compris entre 5 et 15 et C est compris entre 100 et 200.

15. Aluminosilicates cristallins enrichis en silice obtenus par le procédé décrit dans les revendications 1 à 14 caractérisés en ce qu'ils possèdent des rapports Silicium/Aluminium de réseau et global identiques et supérieurs à 10 et en ce que leurs spectres RMN haute résolution de $^{27}$Al ne contiennent que des signaux compris entre 50 et 65 ppm.

16. Application des aluminosilicates selon la revendication 15, comme catalyseurs ou composants de catalyseurs pour les réactions de transformation des hydrocarbures.

17. Application selon la revendication 16 caractérisée en ce que la réaction est l'isomérisation des alcanes, des oléfines et des aromatiques, ou le craquage et l'hydrocraquage ou la polymérisation des oléfines.

18. Application des aluminosilicates selon la revendication 15 comme tamis moléculaires pour les opérations de séchage et de séparation.

## Claims

1. Method for preparation of a crystalline aluminosilicate enriched with silica which has the structure of mazzite, consisting of:

   - preparing a crystallisation gel which contains a source of tetravalent silicon, trivalent aluminium and alkali ion, an organic structuring agent and water;
   - heating the gel in order to obtain crystallisation, then separating, washing and drying the crystals;
   - subjecting the crystals to heat treatment in a humid atmosphere;

- then to etching by a mineral acid;
  characterised in that the source of trivalent aluminium consists of crystals of Y zeolite which are obtained in the form of spheres or botryoids.

2. Method according to claim 1, characterised in that the overall composition of the crystallisation gel corresponds to:

$SiO_2/Al_2O_3$ = 20 - 200
$SiO_2$/alkali ion = 1.2 - 2
structuring agent/alkali ion = 0.01 - 0.1
$H_2O$/alkali ion = 15 - 100.

3. Method according to claim 1 or claim 2 characterised in that before undergoing the heat treatment in a humid atmosphere, the crystals are subjected to calcination at a temperature which is sufficient to break down the organic structuring agent.

4. Method according to any one of claims 1 to 3, characterised in that before undergoing the heat treatment in a humid atmosphere, the alkali cations which are present in the crystals are exchanged by protons or ammonium ions.

5. Method according to any one of claims 1 to 4, characterised in that the source of tetravalent silicon is selected from amongst the silicates, solid silicas, colloidal silicas, gels and xerogels, hydrolysable silicic esters, or diatomites.

6. Method according to any one of claims 1 to 5, characterised in that the alkali ion is sodium or a mixture of sodium and potassium.

7. Method according to any one of claims 1 to 6, characterised in that the organic structuring agent is a tetralkylammonium, and preferably tetramethylammonium.

8. Method according to any one of claims 1 to 7, characterised in that the crystallisation gel is heated to a temperature of between 20 and 200°C, and preferably 100 and 150°C at a pressure which is at least equal to the vapour pressure of the gel, for 4 to 96 hours and preferably 10 to 50 hours.

9. Method according to claim 3, characterised in that the calcination is carried out for 5 minutes to 10 hours at a temperature higher than 450°C, in a flow of nitrogen or air or a mixture of both.

10. Method according to claim 4, characterised in that the alkali cations are exchanged by an aqueous solution of an ammonium salt or a mineral or organic acid.

11. Method according to any one of claims 1 to 10, characterised in that the heat treatment in a humid atmosphere is carried out at a temperature of between 450 and 850°C for a period of 1 minute to a few hours.

12. Method according to any one of claims 7 to 11, characterised in that nitric acid, hydrochloric acid or sulphuric acid are used for the etching by the mineral acid.

13. Method according to claim 12, characterised in that the duration of the acid etching is between 10 minutes and 5 hours, the temperature is between 20 and 100°C, and the concentration of the acid is between 0.01 and 10 N.

14. Method according to any one of claims 1 to 13, characterised in that the Y zeolite in the form of spheres is prepared by hydrothermal crystallisation of a mixture which contains a source of tetravalent silicon, a source of trivalent aluminium, soda and water, in the proportions:
    a $Na_2O$; $Al_2O_3$ ; b $SiO_2$ ; C $H_2O$
    in which a is between 2 and 6, b is between 5 and 15, and C is between 100 and 200.

15. Crystalline aluminosilicates enriched with silica, obtained by the method described in claims 1 to 14, characterised in that they have lattice and overall ratios of silicon / aluminium which are identical and greater than 10, and in that their high-resolution NMR spectra of $^{27}$Al contain signals of between 50 and 65 ppm alone.

16. Application of aluminosilicates according to claim 15, to catalysts or components of catalysts for hydrocarbon transformation reactions.

**17.** Application according to claim 16, characterised in that the reaction is isomerisation of alkanes, olefins and aromatic substances, or cracking and hydrocracking or polymerisation of olefins.

**18.** Application of aluminosilicates according to claim 15, to molecular filters for operations of drying and separation.

**Patentansprüche**

**1.** Verfahren zur Herstellung eines mit Kieselsäure angereicherten kristallinen Alumosilikats der Mazzitstruktur durch

- Herstellung eines Kristallisationsgels, das eine Si(IV)-, eine Al(III)- und eine Alkalimetallionenquelle, ein organisches Strukturierungsmittel und Wasser umfaßt,

- Erwärmung des Gels zur Durchführung der Kristallisation, Abtrennung, Waschen und Trocknung der Kristalle,

- Wärmebehandlung der Kristalle in feuchter Atmosphäre und

- Angriff durch eine Mineralsäure,
  dadurch **gekennzeichnet,** daß die Al(III)-Quelle aus Kristallen des Zeoliths Y in Kugel- oder Traubenform vorliegt.

**2.** Verfahren nach Anspruch 1, dadurch **gekennzeichnet,** daß die Gesamtzusammensetzung des Kristalisationsgels wie folgt ist:

$SiO_2/Al_2O_3$ = 20 - 200
$SiO_2$/Alkalimetallion = 1,2 - 2
Strukturierungsmittel/Alkalimetallion = 0,01 - 0,1
$H_2O$/Alkalimetallion = 15 - 100.

**3.** Verfahren nach Anspruch 1 oder 2, dadurch **gekennzeichnet,** daß vor der Wärmebehandlung in feuchter Atmosphäre die Kristalle bei einer für die Zersetzung des organischen Strukturierungsmittels ausreichend hohen Temperatur geglüht werden.

**4.** Verfahren nach einem der Ansprüche 1 bis 3, dadurch **gekennzeichnet,** daß vor der Wärmebehandlung in feuchter Atmosphäre die in den Kristallen enthaltenen Alkalimetallkationen durch Protonen oder Ammoniumionen ausgetauscht werden.

**5.** Verfahren nach einem der Ansprüche 1 bis 4, dadurch **gekennzeichnet,** daß die Si(IV)-Quelle unter Silikaten, festen Kieselsäuren, kolloidalen Kieselsäuren, Gelen und Xerogelen, hydrolysierbaren Kieselsäureestern oder Diatomiten ausgewählt wird.

**6.** Verfahren nach einem der Ansprüche 1 bis 5, dadurch **gekennzeichnet,** daß das Alkalimetallion Natrium oder ein Gemisch aus Natrium und Kalium ist.

**7.** Verfahren nach einem der Ansprüche 1 bis 6, dadurch **gekennzeichnet,** daß das organische Strukturierungsmittel ein Tetraalkylammonium und vorzugsweise Tetramethylammonium ist.

**8.** Verfahren nach einem der Ansprüche 1 bis 7, dadurch **gekennzeichnet,** daß das Kristallisationsgel auf eine Temperatur von 20 bis 200°C und vorzugsweise von 100 bis 150°C, unter einem Druck, der wenigstens der Dampfspannung des Gels entspricht, während 4 bis 96 Stunden und vorzugsweise 10 bis 50 Stunden erwärmt wird.

**9.** Verfahren nach Anspruch 3, dadurch **gekennzeichnet,** daß das Glühen während 5 Minuten bis 10 Stunden bei einer Temperatur von über 450°C unter einem Strom aus Stickstoff oder Luft oder einem Gemisch davon durchgeführt wird.

**10.** Verfahren nach Anspruch 4, dadurch **gekennzeichnet,** daß die Alkalimetallkationen durch eine wäßrige Lösung eines Ammoniumsalzes oder einer Mineralsäure oder organischen Säure ausgetauscht werden.

**11.** Verfahren nach einem der Ansprüche 1 bis 10, dadurch **gekennzeichnet,** daß die Wärmebehandlung in feuchter Atmosphäre bei einer Temperatur von 450 bis 850°C während einer Minute bis mehrerer Stunden durchgeführt wird.

12. Verfahren nach einem der Ansprüche 7 bis 11, dadurch **gekennzeichnet,** daß für den Angriff durch die Mineralsäure Salpeter-, Salz- oder Schwefelsäure verwendet wird.

13. Verfahren nach Anspruch 12, dadurch **gekennzeichnet,** daß die Dauer des Säureangriffs 10 Minuten bis 5 Stunden, die Temperatur 20 bis 100°C und die Säurekonzentration 0,01 bis 10 N betragen.

14. Verfahren nach einem der Ansprüche 1 bis 13, dadurch **gekennzeichnet,** daß der in Kugelform vorliegende Zeolith durch hydrothermale Kristallisation eines Gemisches hergestellt wird, das eine Si(IV)-Quelle, eine Al(III)-Quelle, Natriumhydroxid und Wasser in den folgenden Verhältnissen enthält:
a $Na_2O$; $Al_2O_3$; b $SiO_2$; C $H_2O$,
worin a einen Wert von 2 bis 6, b einen Wert von 5 bis 15 und C einen Wert von 100 bis 200 bedeuten.

15. Nach dem in den Ansprüchen 1 bis 14 beschriebenen Verfahren hergestellte mit Kieselerde angereicherte kristalline Alumosilikate, dadurch **gekennzeichnet,** daß sie identische Si:Al-Verhältnisse im Gitter und Gesamtverhältnisse von über 10 aufweisen und ihre Spektren der hochauflösenden $^{27}$Al-NMR-Spektroskopie nur Signale zwischen 50 und 65 ppm aufweisen.

16. Verwendung der Alumosilikate nach Anspruch 15 als Katalysatoren oder als Katalysatorkomponenten für Reaktionen zur Umwandlung von Kohlenwasserstoffen.

17. Verwendung nach Anspruch 16, dadurch **gekennzeichnet,** daß die Reaktion die Isomerisierung von Alkanen, Olefinen oder Aromaten, das Kracken und das Hydrokracken oder die Polymerisation von Olefinen ist.

18. Verwendung der Alumosilikate nach Anspruch 15 als Molekularsiebe für Trocknungs- und Trennungsprozesse.

S4R

S6R

200    150    100    50    0    -50
PPM